(19) 

Europäisches
Patentamt
European
Patent Office
Office européen
des brevets

(11) **EP 4 781 895 A1**

(12) **EUROPEAN PATENT APPLICATION**
published in accordance with Art. 153(4) EPC

(43) Date of publication:
**29.07.2026 Bulletin 2026/31**

(21) Application number: **24868189.2**

(22) Date of filing: **11.09.2024**

(51) International Patent Classification (IPC):
**A61B 3/10** (2006.01)    **G01N 21/17** (2006.01)

(52) Cooperative Patent Classification (CPC):
**A61B 3/10; G01N 21/17**

(86) International application number:
**PCT/JP2024/032581**

(87) International publication number:
**WO 2025/063112 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.09.2023 JP 2023159176**

(71) Applicant: **Nikon Corporation
Tokyo 140-8601 (JP)**

(72) Inventors:
• **SHIRAISHI, Natsumi
Tokyo 140-8601 (JP)**
• **LI, Yongbo
Tokyo 140-8601 (JP)**
• **KASAI, Hiroshi
Tokyo 140-8601 (JP)**

(74) Representative: **Hoffmann Eitle
Patent- und Rechtsanwälte PartmbB
Arabellastraße 30
81925 München (DE)**

(54) **SIGNAL PROCESSING METHOD FOR OPHTHALMOLOGIC APPARATUS, OPHTHALMOLOGIC APPARATUS, AND PROGRAM**

(57)    A signal processing method performed by a processor of an ophthalmic device including a step of obtaining an interference signal between measurement light and reference light, a step of obtaining a peak value component determined by a distribution of brightness in the interference signal, a step of obtaining a corrected interference signal by subtracting a signal of magnitude determined from the peak value component from the interference signal, a step of employing the corrected interference signal as the interference signal, and repeatedly executing the step of obtaining the peak value component for the interference signal and the step of obtaining the corrected interference signal until the subtraction result corrected interference signal is a predetermined threshold or lower, and a step of obtaining OCT data based on the corrected interference signal of the threshold or lower.

FIG.6

START

S102 ACQUIRE INTERFERENCE SPECTRUM

S104 PERFORM RE-SAMPLING, ZERO PADDING, WINDOW FUNCTION PROCESSING

S106 COMPUTE MEAN VALUES OF LIGHT INTENSITIES OF INITIAL INTERFERENCE PATTERN OF ALL A-SCANS AND COMPUTE THRESHOLD

INSTRUCT A-SCAN

S108

S110 PERFORM DISPERSION CORRECTION

S112 PERFORM INVERSE FAST FOURIER TRANSFORM (IFFT)

S114 PERFORM MULTI PEAK DETECTION (MPD)

S116 SAVE DETECTED MULTI PEAK VALUES

S118 PERFORM FAST FOURIER TRANSFORM (FFT)

S120 PERFORM INVERSE DISPERSION CORRECTION

S122 EXTRACT REAL PART

S124 SUBTRACT MPD COMPONENTS FROM INTERFERENCE SPECTRUM

S126 LIGHT INTENSITY I ≤ TH    N

S128 ALL A-SCANS COMPLETED?    N

Y

END

EP 4 781 895 A1

**Description**

Technical Field

Technology disclosed herein relates to a signal processing method for an ophthalmic device, an ophthalmic device, and a program.

Background Art

Among devices for observing an examined eye, optical coherence tomography (OCT) devices are known that acquire tomographic images of the examined eye (see, for example, specification of US Patent Publication No. 2020/0037872). In OCT devices, there is a demand to acquire tomographic images of the examined eye with high precision over wider ranges.

SUMMARY OF INVENTION

Technical Problem

A first aspect of technology disclosed herein is an ophthalmic device including a processor, the processor in the ophthalmic device is configured to execute processing including:

a step of obtaining an interference signal between measurement light and reference light;
a step of obtaining a peak value component determined by a distribution of brightness in the interference signal;
a step of obtaining a corrected interference signal by subtracting a signal of magnitude determined from the peak value component from the interference signal;
a step of employing the corrected interference signal as the interference signal, and repeatedly executing the step of obtaining the peak value component for the interference signal and the step of obtaining the corrected interference signal until the subtraction result corrected interference signal is a predetermined threshold or lower; and
a step of obtaining OCT data based on the corrected interference signal of the threshold or lower.

A second aspect of technology disclosed herein is a signal processing method performed by a processor of an ophthalmic device, the signal processing method of the ophthalmic device includes:

a step of obtaining an interference signal between measurement light and reference light;
a step of obtaining a peak value component determined by a distribution of brightness in the interference signal;
a step of obtaining a corrected interference signal by subtracting a signal of magnitude determined from the peak value component from the interference signal;
a step of employing the corrected interference signal as the interference signal, and repeatedly executing the step of obtaining the peak value component for the interference signal and the step of obtaining the corrected interference signal until the subtraction result corrected interference signal is a predetermined threshold or lower; and
a step of obtaining OCT data based on the corrected interference signal of the threshold or lower.

A third aspect of technology disclosed herein is a program that causes processing to be executed on a computer, the processing including:

a step of obtaining an interference signal between measurement light and reference light;
a step of obtaining a peak value component determined by a distribution of brightness in the interference signal;
a step of obtaining a corrected interference signal by subtracting a signal of magnitude determined from the peak value component from the interference signal;
a step of employing the corrected interference signal as the interference signal, and repeatedly executing the step of obtaining the peak value component for the interference signal and the step of obtaining the corrected interference signal until the subtraction result corrected interference signal is a predetermined threshold or lower; and
a step of obtaining OCT data based on the corrected interference signal of the threshold or lower.

BRIEF DESCRIPTION OF DRAWINGS

Fig. 1 is a block diagram illustrating a configuration of an ophthalmic system according to an exemplary embodiment.

Fig. 2 is a block diagram illustrating a hardware configuration of an ophthalmic device according to an exemplary embodiment.

Fig. 3 is a block diagram illustrating a hardware configuration of an OCT unit according to an exemplary embodiment.

Fig. 4 is a functional block diagram illustrating an ophthalmic device according to an exemplary embodiment.

Fig. 5 is a flowchart illustrating a flow of signal processing in an ophthalmic device according to an exemplary embodiment.

Fig. 6 is a flowchart illustrating a flow of full-range OCT processing in an ophthalmic device according to an exemplary embodiment.

Fig. 7 is a flowchart illustrating processing to derive a coefficient related to a threshold.

Fig. 8 is a diagram illustrating an example of tomographic images of a fundas.

Fig. 9 is a diagram illustrating an example of a tomographic image in a case in which full-range OCT processing has been executed.

Fig. 10 is a diagram illustrating a tomographic image in a case in which normal full-range OCT processing has been executed.

Fig. 11 is a conceptual diagram related to OCT images (tomographic images) from full-range OCT processing.

Fig. 12 is a conceptual diagram of how an OCT image (tomographic image) changes according to a number of times of repetition of an FR process.

Fig. 13 is a diagram illustrating a screen displayed on a display.

DESCRIPTION OF EMBODIMENTS

Detailed description follows regarding an exemplary embodiment to implement technology disclosed herein, with reference to the drawings. Note that configuration elements and processing carrying out the same operation and function are appended with the same reference numerals across the drawings, and sometimes duplicate description thereof is omitted as appropriate. Moreover, sometimes description will be omitted for configuration not directly related to the present disclosure and for known configuration. The dimensions and proportions in the drawings may be exaggerated for ease of explanation, and sometimes differ from actual proportions. Furthermore, each of the drawings is merely illustrated schematically to such a degree as to enable sufficient understanding of technology disclosed herein. Technology disclosed herein is accordingly not limited to the examples illustrated.

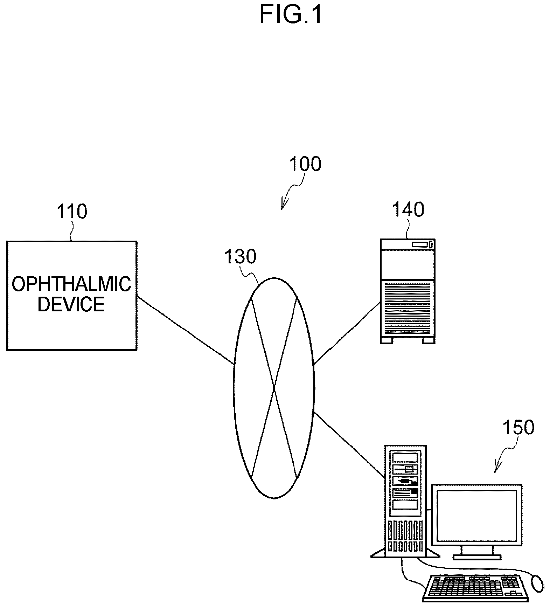

Description follows regarding a configuration of an ophthalmic system 100 according to an exemplary embodiment of technology disclosed herein, with reference to Fig. 1. Fig. 1 is a block diagram illustrating a configuration an ophthalmic system 100 according to the present exemplary embodiment. As illustrated in Fig. 1, the ophthalmic system 100 includes an ophthalmic device 110, a network 130, a management server device (hereafter referred to as "management server") 140, and an image display device (hereafter referred to as "image viewer") 150.

The ophthalmic device 110, the management server 140, and the image viewer 150 are connected together through the network 130. The network 130 is a freely selected network such as a LAN, WAN, the internet, a wide area Ethernet, or the like. For example, a LAN may be employed as the network 130 in cases in which the ophthalmic system 100 is built in a single hospital.

The ophthalmic device 110 is a device for imaging an examined eye, and executes at least one of examination, measurement, or treatment related to ophthalmology.

The management server 140 receives examined eye images obtained by imaging the examined eye with the ophthalmic device 110 associated with patient ID over the network 130, and then stores them in a non-illustrated memory. The memory of the management server 140 is accordingly stored with examined eye images corresponding to patient IDs of each patient. Moreover, the management server 140 transmits the examined eye images to the image viewer 150 over the network 130.

The image viewer 150 is a touch panel display or the like, and includes a communication function. The image viewer 150 displays examined eye images acquired by the management server 140.

Note that other ophthalmic equipment (examination equipment for measuring a field of view, measuring intraocular pressure, or the like) and/or a diagnostic support device that analyzes images using artificial intelligence may be connected to the ophthalmic device 110, the management server 140, and the image viewer 150 over the network 130.

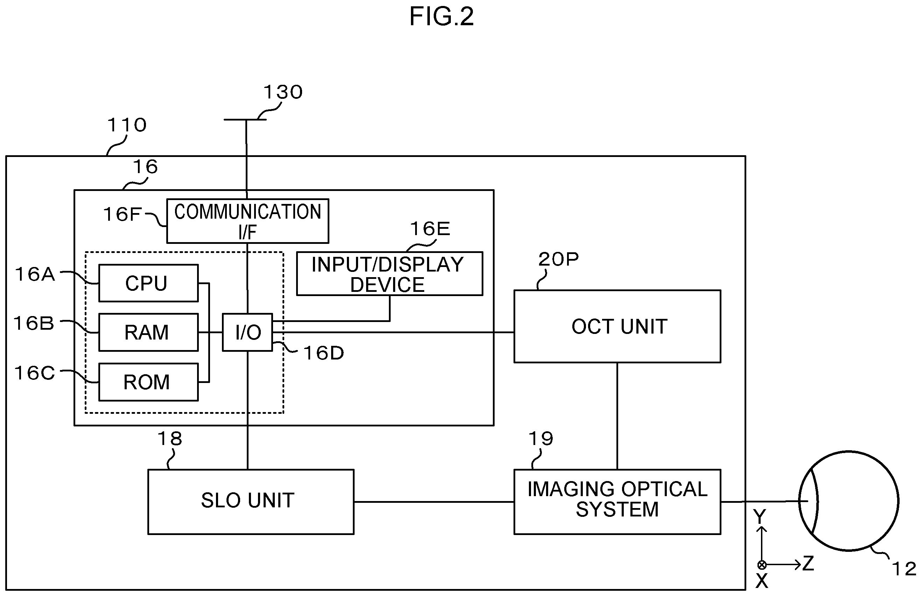

Next, explanation follows regarding a configuration of the ophthalmic device 110, with reference to Fig. 2. Fig. 2 is a block diagram illustrating a hardware configuration of the ophthalmic device 110 according to the present exemplary embodiment. As illustrated in Fig. 2, the ophthalmic device 110 includes a control device 16, a scanning laser ophthalmoscope (SLO) unit 18, an optical coherence tomography (OCT) unit 20P, and an imaging optical system 19. The control device 16 controls the SLO unit 18, the OCT unit 20P, and the imaging optical system 19. The control device 16 controls the SLO unit 18 and the imaging optical system 19, and acquires an image of the fundus of an examined eye 12, or an image of an anterior eye portion thereof. The control device 16 controls the OCT unit 20P and the imaging optical system 19, and acquires a tomographic image of the fundus or the anterior eye portion of the examined eye 12.

With the ophthalmic device 110 installed on a horizontal plane and a horizontal direction taken as an "X direction", a direction perpendicular to the horizontal plane is denoted a "Y direction", and an optical axis direction of the imaging optical system 19 is denoted as a "Z direction". The device is arranged with respect to the examined eye 12 such that a center of the pupil of the examined eye is positioned in the direction of the optical axis. The X direction, the Y direction, and the Z direction are thus mutually perpendicular directions.

In cases in which one-dimensional data is to be acquired in the depth direction, one-dimensional OCT data (called "A-scan data") is obtained by scanning a single point on the fundus in the depth (optical axis) direction (called an "A-scan"). In cases in which two-dimensional data is to be acquired, A-scans are obtained plural times while moving the scan position in a direction orthogonal to the depth direction (called a "B-scan") and two-dimensional OCT data (called "B-scan data") is obtained. In cases in which three-dimensional data is to be acquired, B-scans are repeated while moving the scan position in a direction orthogonal to the B-scan direction (called a "C-scan"), and OCT data (called "volume scan data") is obtained. Three-dimensional OCT data is generated from the volume scan data, and two-dimensional en-face images and the like are generated based on the three-dimensional OCT data.

The control device 16 includes a computer provided with a Central Processing Unit (CPU) 16A, Random Access Memory (RAM) 16B, Read-Only Memory (ROM) 16C, an input/output (I/O) port 16D, an input/display device 16E, and a communication interface (I/F) 16F. Each configuration of the control device 16 is connected so as to be able to communicate with each other through the bus.

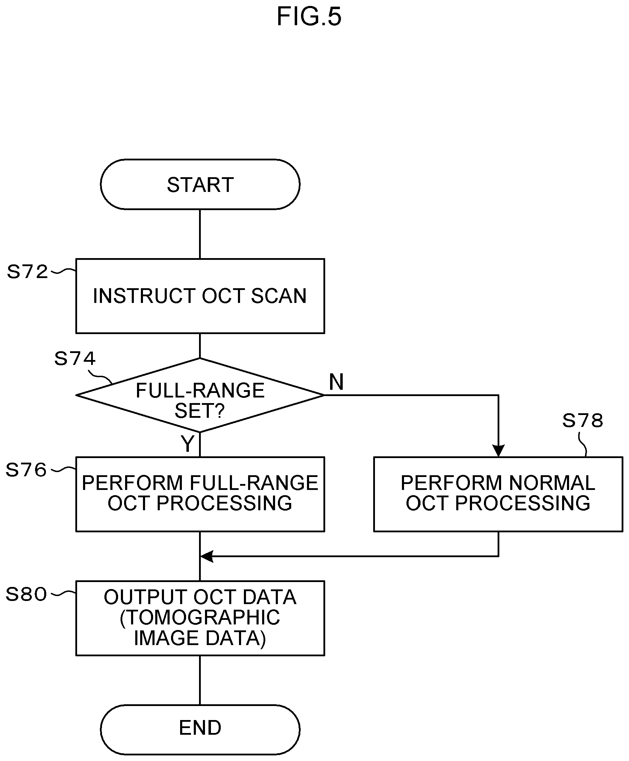

The CPU 16A is a central processing unit and executes various programs and controls each section. Namely, the CPU 16A reads a program from the ROM 16C, and executes the program using the RAM 16B as a workspace. The CPU 16A controls each configuration and performs various computation processing according to the program stored in the ROM 16C. In the present exemplary embodiment, a signal processing program for a processing the light intensity distribution (interference spectrum) of interference light as illustrated in Fig. 5, described later, is stored on the ROM 16C.

The CPU 16A is an example of a "processor" of technology disclosed herein. The RAM 16B and the ROM 16C are each an example of a storage section for storing information. The control device 16 illustrates an example of a computer.

The RAM 16B serves as a workspace for temporarily storing programs and data. The ROM 16C stores various programs and various data.

Note that the control device 16 may be configured further including storage configured by a storage medium such as a hard disk drive (HDD), solid state drive (SSD), or the like. In such cases, various programs including an operating system and various data may be stored in the storage. The signal processing program illustrated in Fig. 5 may be stored in the storage instead of in the ROM 16C.

The input/display device 16E is connected to the CPU 16A through an I/O 16D. The input/display device 16E includes a graphical user interface (GUI) for displaying images of the examined eye 12 and receiving various instructions from a user. An example of the GUI is a touch panel display.

The control device 16 is connected to the network 130 through the communication interface 16F. The communication interface 16F is an interface for communicating with other devices and, for example, employs a standard such as Ethernet (registered trademark), FDDI, or Wi-Fi (registered trademark).

Although in Fig. 2 the control device 16 of the ophthalmic device 110 includes the input/display device 16E, technology disclosed herein is not limited thereto. For example, a configuration may be adopted in which the control device 16 of the ophthalmic device 110 is not provided with the input/display device 16E, and instead a separate input/display device is provided that is physically independent of the ophthalmic device 110. In such cases the display device includes an image processing processor unit operating under control of the CPU 16A of the control device 16. The image processing processor unit may be configured so as to display acquired images and the like based on an image signal instructed for output by the CPU 16A.

In the following, front-view images of the retina generated based on SLO data acquired by the SLO unit 18 are called SLO images, and tomographic images, en-face images, and the like of the retina generated based on OCT data acquired by the OCT unit 20P are called OCT images. Note that SLO images are sometimes referred to as being two-dimensional fundus images. Moreover, OCT images are sometimes referred to as fundus tomographic images, posterior eye portion tomographic images, and anterior eye portion tomographic images, depending on the imaging site on the examined eye 12.

The angle of view of the ophthalmic device 110 is preferably a wide angle, and ultrawide angles, as described later, are preferable. In such cases, the obtained images may be called ultra wide field (UWF) fundus images. Images from imaging the fundus at an ultra-wide angle are hereinafter called UWF fundus images.

Observations of the fundus with a wide field of view (FOV) are implemented by the imaging optical system 19. The FOV indicates a range capable of being imaged by an imaging device. The FOV may be expressed as a viewing angle. In the present exemplary embodiment, the viewing angle may be defined in terms of an internal illumination angle and an external illumination angle. The external illumination angle is the angle of illumination by a light beam shone from the ophthalmic device 110 toward the examined eye 12, and is an angle of illumination defined with respect to the pupil. The internal illumination angle is the angle of illumination of a light beam shone onto the fundus, and is an angle of illumination defined with respect to an eyeball center. Correspondence relationships exist between the external illumination angle and the internal illumination angle. For example, an external illumination angle of 120° is equivalent to an internal illumination angle of about 160°. The internal illumination angle in the present exemplary embodiment is 200°.

An SLO fundus image obtained by imaging at an imaging angle of view of an internal illumination angle of 160° or greater is called a UWF-SLO fundus image (UWF fundus image).

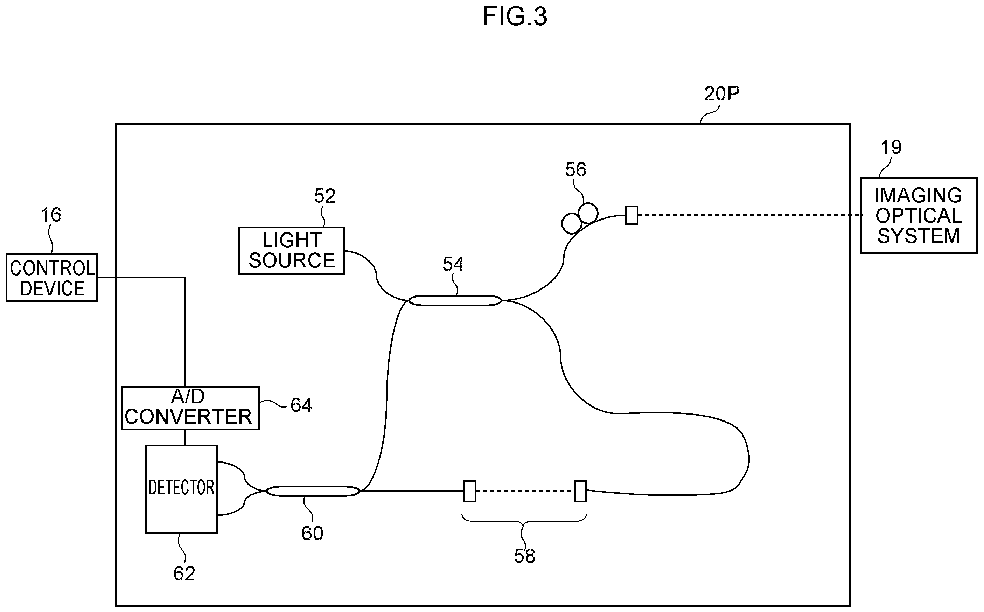

Fig. 3 is a block diagram illustrating a hardware configuration of the OCT unit 20P according to the present exemplary embodiment. As illustrated in Fig. 3, the OCT unit 20P includes a light source 52, a first light coupler 54, a polarization adjustment section 56, a delay-line 58, a second light coupler 60, a detector 62, and an analogue-digital (A/D) converter 64.

The light source 52 emits light for optical coherence tomographic imaging. The light source 52 is a wavelength-swept light source that outputs wavelength-swept light in which a central wavelength of emitted light is rapidly changed by sweeping a specific wavelength range at a specific wavelength sweep speed. The light source 52 includes, for example, a near infrared variable-wavelength laser. The OCT unit 20P accordingly acquires OCT data of the fundus using the principle of swept source-optical coherence tomography (SS-OCT).

Note that the OCT unit 20P is not limited to being an SS-OCT using a wavelength-swept light source as the light source 52. For example, an SD-OCT using a super luminescent diode (SLD) may be employed.

The first light coupler 54 divides the path of light emitted from the light source 52 into two paths. One of the paths is a path of light toward the polarization adjustment section 56, and the other path is for light toward the delay-line 58.

Note that although in Fig. 3 an example is illustrated in which the polarization adjustment section 56 is arranged on one path from out of the two paths branched by the first light coupler 54 for the path of light from the light source 52, the present disclosure is not limited thereto. Namely, the polarization adjustment section 56 may be arranged on at least one path from out of the two paths branched (divided) by the first light coupler 54. This is because any configuration capable of matching two polarized light states to the same oscillation orientation (for example, maximizing the interference state) using polarization adjustment section(s) 56 is suitable when combining and interfering measurement light and reference light using the second light coupler 60. Moreover, although in Fig. 3 an example is illustrated in which the delay-line 58 is arranged on one path from out of the two paths, the present disclosure is not limited thereto. Namely, the delay-line 58 may be arranged on at least one path from out of the two paths branched by the first light coupler 54. This is because any configuration capable of matching light path lengths of two lights to the same light path length using delay-line(s) 58 when combining and interfering measurement light and reference light using the second light coupler 60 is suitable.

The polarization adjustment section 56 adjusts the polarized light state, which is the orientation of oscillation of light oscillating in a specific spatial orientation. The light whose polarized state has been adjusted is introduced into the imaging optical system 19.

The light introduced into the imaging optical system 19 is scanned in an X-direction and/or Y direction by the imaging optical system 19. The scanning light serves as measurement light and is illuminated onto the fundus via the pupil of the examined eye 12. The scanning light is scanned, and so each scan time is associated with a respective scan position on the fundus. The scanning speed is a speed that enables acquisition of B-scan data by B-scan at each scan position. Note that the scanning speed and the light source sweep speed are different in the X or Y direction, with the sweep speed being the A-scan speed for acquiring an A-scan signal at a single point. The measurement light reflected at the fundus is introduced into the OCT unit 20P via the imaging optical system 19, and is introduced into the second light coupler 60 via the polarization adjustment section 56 and the first light coupler 54.

The other light emitted from the light source 52 and branched by the first light coupler 54 serves as the reference light introduced into the delay-line 58, and introduced into the second light coupler 60 via the delay-line 58.

As described above, the delay-line 58 makes the light path length of the reference light and the light path length of the measurement light the same. These light path lengths depend on the eye axial length, and the delay-line 58 can be adjusted by an operator, for example prior to starting operation of the scanning light while looking at a fundus image such that the tomographic image is positioned at an appropriate range.

The measurement light (return light) reflected by the examined eye 12, and the reference light, are combined by the second light coupler 60 and generate interference light. The detector 62 detects the interference light, removes noise, and outputs a detection signal. The detection signal indicates, for example, a power distribution of interference light, such as brightness or the like, namely, a light intensity distribution of interference light (hereinafter called an interference spectrum). The A/D converter 64 performs A/D conversion on the interference spectrum from the detector 62 and outputs to the control device 16. The interference spectrum is stored in the RAM 16B of the control device 16.

Note that in cases in which an SD-OCT is employed as the OCT unit 20P, the interference light is separated for each wavelength component and detected by arranging a spectroscope at the position of the detector 62, and arranging an image grabber board at the position of the A/D converter 64.

The management server 140 and the image viewer 150 each include a computer, an input device, an external storage device, a display device, a communication interface, and the like.

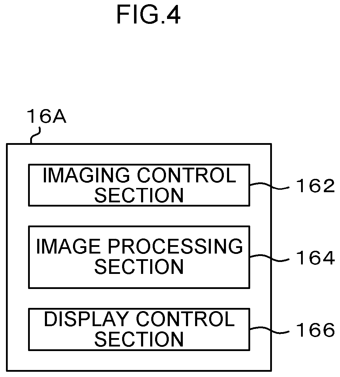

As illustrated in Fig. 4, the CPU 16A of the ophthalmic device 110 of the present exemplary embodiment functions as an imaging control section 162, an image processing section 164, and a display control section 166.

The image processing section 164 is an example of an "interference signal acquisition section", "component acquisition section", "correction interference signal acquisition section", "repeat execution section", and "OCT signal acquisition section" of technology disclosed herein.

Various images (for example, images called artifacts) appear in fundus images of the examined eye acquired by OCT. For example, a complex conjugate component image is generated as a mirror image in a fundus image acquired by full-range OCT (FR-OCT) to expand the observable depth of OCT. The mirror image is a virtual image appearing on the opposite side to the real image with respect to a position where the light path length of measurement light illuminated onto the fundus is the same as the light path length of the reference light. A tomographic image of the examined eye can be acquired over a wider range by forming a tomographic image from the real image appearing at one side (for example, the plus side in the depth direction of the fundus) with respect to the position where the light path length of the measurement light and the light path length of the reference light are the same. Namely, a tomographic image of the fundus can be acquired at full-range by forming a tomographic image by removing a mirror image (an image that is a virtual image appearing in the opposite direction in the depth direction of the fundus, this being the minus side) from the OCT image.

Thus in the present exemplary embodiment, signal processing is performed on the signal obtained using OCT imaging to acquire a tomographic image that accurately reflects the content of the fundus after removing mirror images and the like.

Namely, detailed description follows regarding signal processing on the interference spectrum by the CPU 16A of the ophthalmic device 110, with reference to Fig. 5. The signal processing (signal processing method) illustrated by the flowchart of Fig. 5 is implemented by the CPU 16A of the ophthalmic device 110 executing the signal processing program.

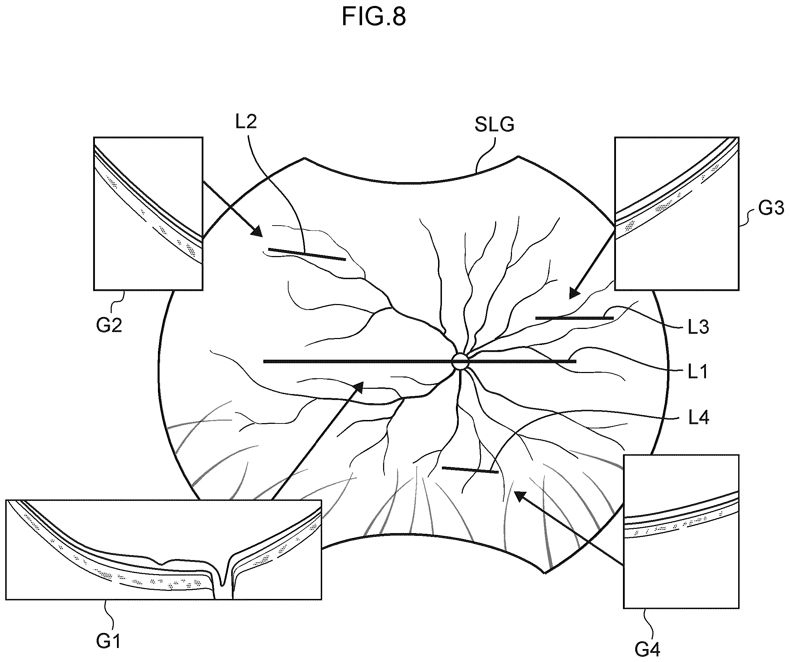

The SLO unit 18 and the imaging optical system 19 are controlled, and an image of the fundus of the examined eye 12 is acquired. The display control section 166 displays a fundus image (also called a UWF-SLO fundus image) SLG as illustrated in Fig. 8 on the input/display device 16E.

The operator checks the fundus image SLG displayed on the input/display device 16E, and uses the input/display device 16E to set a range to acquire a tomographic image. For example, a range L1 is a comparatively wide range including a central portion of the fundus including the optic nerve head (a point (fundus center) where the optical axis of the ophthalmic device 110 intersects with the fundus). Ranges L2, L3, L4 are ranges of peripheral portions at the periphery of the central portion of the fundus.

There is no limit to using an SLO image to set the range for acquiring the tomographic image. For example, a position to acquire a tomographic image may be decided using a two-dimensional en-face image based on OCT data, and a tomographic image may be acquired thereat.

Note that although ranges from L1 to L4 are each a line segment in a horizontal direction of the fundus (B-scan direction), the range for acquiring a tomographic image is not limited to line segments in the B-scan direction, and may be line segments in a direction intersecting with the B-scan direction, and may be a rectangular range.

Moreover, although described in detail later, a user may pre-select whether to execute full-range OCT processing or to execute normal OCT processing, and which processing is to be executed may be set in the ophthalmic device 110. Moreover, sometimes a user might wish to perform full-range OCT processing after executing normal OCT processing. In such cases, a time series may be selectable to enable execution of full-range OCT processing after executing normal OCT processing.

In this manner, an operator sets the range for acquiring a tomographic image and the processing to be executed (at least one processing from out of normal OCT processing or full-range OCT processing), instructs "start" using the input/display device 16E, and starts running the signal processing program.

At step S72, the imaging control section 162 instructs the OCT unit 20P and the imaging optical system 19 to perform OCT scanning to so as to acquire a tomographic image of the set range.

At step S74, the imaging control section 162 determines whether or not the imaging control section 162 has been set so as to output a tomographic image at full-range to a display screen of the input/display device 16E. Reference here to full-range means that the output range of the image output to the display screen is a range (positive region) including a range in the depth direction of the fundus with reference to a position corresponding to the light path length of the reference light in the examined eye 12, and a range (negative region) in the opposite direction to the depth direction.

In cases in which full-range is not set, a tomographic image is displayed with the output range of the tomographic image for output to the display screen being a range in the depth direction of the fundus with reference to the position corresponding to the light path length of the reference light in the examined eye 12, and is displayed in a fixed range not including the reference position and where a mirror image does not appear. Fig. 8 is an example of tomographic images displayed when full-range is not set, and illustrates display of examples of respective tomographic images from G1 to G4 corresponding to the ranges from L1 to L4.

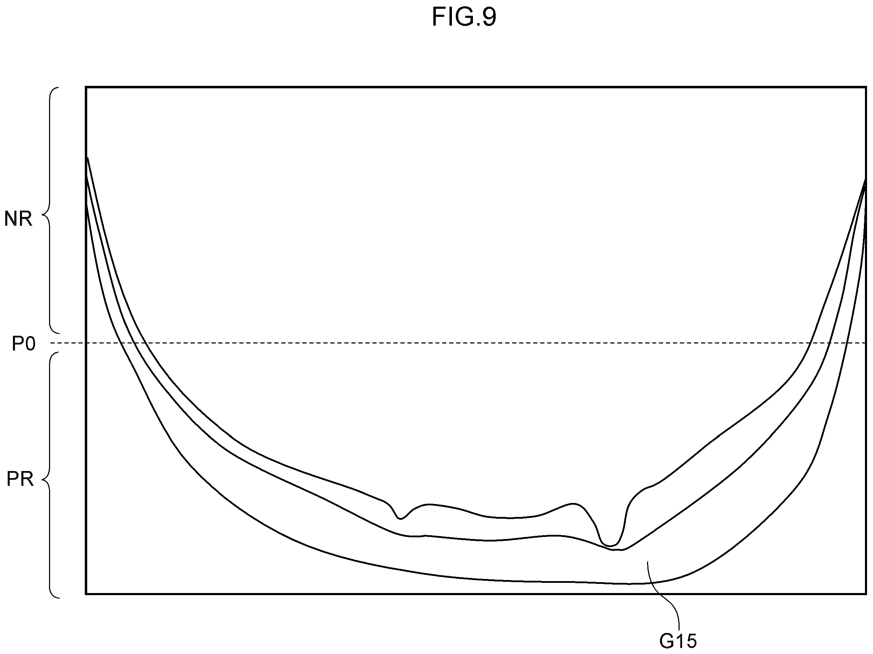

In cases in which full-range is set, the tomographic image is displayed with an output range of the tomographic image for output to the display screen being a range in the depth direction of the fundus with reference to the position corresponding to the light path length of the reference light in the examined eye 12 and in a range in the opposite direction to the depth direction. Fig. 9 illustrates a display example of a tomographic image G15 corresponding to the range L1 displayed when full-range has been set. As illustrated in Fig. 9, when full-range has been set, the tomographic image G15 is displayed with the output range of the image output to the display screen as a range including a range (positive region) PR in the depth direction of the fundus with reference to the position PO corresponding to the light path length of the reference light in the examined eye 12 and a range (negative region) NR in the opposite direction to the depth direction. This means that in cases in which full-range is set, wider tomographic images can be displayed than cases in which full-range is not set.

The full-range OCT processing (step S76) performed when full-range is set takes a longer processing time than the normal OCT processing (step S78) when the full-range is not set. A user pre-selects whether to execute full-range OCT processing or to execute normal OCT processing, and sets this on the ophthalmic device 110. At step S74, determination is made as to which processing has been set by the user.

The processing of step S74 is not limited to processing to determine whether the processing set in the ophthalmic device 110 by the user is full-range OCT processing or normal OCT processing.

In cases in which full-range has been set, a wider tomographic image can be displayed than cases in which full-range has not been set, as described above. Thus the processing of step S74 may be configured so as to determine whether or not the length in a specific direction of the range for acquiring tomographic images as set by the user is longer than a predetermined length. In such cases, affirmative determination is made at step S74 when the length in the specific direction of the range for acquiring tomographic images as set by the user is longer than the predetermined length. Negative determination is made at step S74 when the length in the specific direction of the range for acquiring tomographic images as set by the user is not determined to be longer than the predetermined length.

A configuration may be adopted such that determination is made as to whether or not a position of at least part of the range for acquiring tomographic images is positioned in a range of peripheral portions at the periphery of the central portion of the fundus. For example, affirmative determination may be made at step S74 when the position of at least part of the range for acquiring tomographic images is positioned in the range of the peripheral portions at the periphery of the central portion of the fundus. However, negative determination may be made at step S74 when the position of at least part of the range for acquiring tomographic images is not positioned in the range of the peripheral portions at the periphery of the central portion of the fundus.

When negative determination is made at step S74, the image processing section 164 executes normal OCT processing at step S78. When the processing of step S78 has been completed, the signal processing proceeds to step S80. Note that the normal OCT processing includes various processing, however detailed explanation thereof will be omitted because this is known processing.

When affirmative determination is made at step S74, the image processing section 164 executes full-range OCT processing at step S76.

As described above, in cases in which full-range OCT processing is selectable after normal OCT processing has been executed, determination processing to determine whether to execute full-range OCT processing may be inserted after the processing of step S78, with processing transitioning to step S76 if affirmative determination is made, and processing transitioning to the processing of step S80 if negative determination is made.

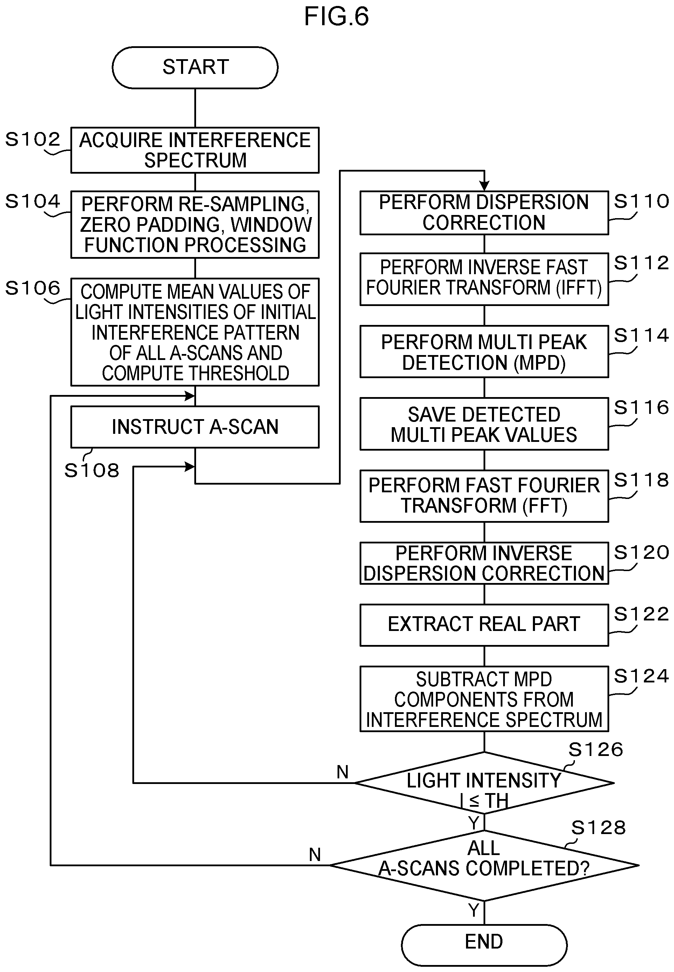

Next, description follows regarding full-range OCT processing, with reference to Fig. 6.

At step S102, the image processing section 164 acquires the interference spectrum stored in the RAM 16B of the control device 16. The interference spectrum r1, r2, ..., rn is data of light intensities for each wavelength component in the wavelength sweep range at each of the scan positions corresponding to each scan time t1, t2, ..., tn. Namely, data representing a spectrum distribution of wavelengths and light intensities of an interference pattern obtained for each A-scan.

The interference spectrum r1, r2, ..., rn is an example of an "interference signal" of technology disclosed herein.

Note that when SD-OCT is employed in the OCT unit 20P, the interference spectrum stored in the control device 16 is data of the light intensities of each wavelength component obtained by the detector 62, which is a spectroscope, at each scan position corresponding to each scan time.

At step S104, the image processing section 164 executes pre-processing on each of the interference spectra to generate a more vivid tomographic image. The pre-processing is, for example, re-sampling, zero padding, window function processing, and the like. Because the sampling intervals when the interference spectra are obtained are not constant and are sparse, re-sampling is processing to make the sampling intervals uniform by signal processing. Zero padding is processing to improve the definition of the tomographic images compared to when such processing is not performed by increasing the number of data points. Specifically, in cases in which the numerical values of the data are integers, this is processing to fill the left side of the numerical values with zeros. For example, when a 10 digit representation is desired, if the numerical value of the data is 12345, then this is made 0000012345. Window function processing is processing to weight data right at the center of each interference spectra with a greater weight than data at the periphery thereof. Note that zero padding may be omitted.

At step S106, the image processing section 164 computes mean values of light intensities of an initial interference pattern of all A-scans, and computes a threshold therefrom. The threshold computed at step S106 is an example of a threshold th for determining whether or not to repeat processing in a full-range process (hereafter referred to as a FR process) executed for each A-scan, described later. The FR process is a process to remove a signal component of a complex conjugate component image arising as a mirror image, namely, a process to remove signal components of a mirror image (hereafter referred to as mirror signal) from the signal representing the interference spectrum. Detailed description is given later.

The processing of step S106 is an example of processing to derive the "threshold" of technology disclosed herein.

The image processing section 164 computes mean values of all data related to the light intensities of A-scans used in an image formed by a B-scan (for example, a fundus tomographic image), and takes this as the mean value of light intensity for an initial interference pattern (interference spectra) of all A-scans. The image processing section 164 derives the threshold th from the mean value. The following Equation (1) illustrates an example of the threshold th (a condition equation). In the equation, f is a light intensity of an initial interference spectrum (interference pattern or interference signal), and M is a total number of the A-scans contained in the B-scan. m is a variable, and E is a predetermined coefficient.

$$\mathrm{th} = E * \frac{\sum_{m=1}^{M} \|f_m\|^2}{M} \quad \cdots (1)$$

At step S108, the image processing section 164 specifies one A-scan (for example, a first-most A-scan of the B-scan direction) of all the A-scans, and executes the FR process. In the example illustrated in Fig. 6, the processing from step S110 to step S124 is executed as the FR process.

At step S110, the image processing section 164 executes known dispersion correction processing to perform dispersion correction numerically on the interference spectrum resulting from pre-processing at step S104. The components of light dispersion caused by the optical system of the OCT unit 20P of the ophthalmic device 110 are able to be dispersion corrected numerically by this dispersion correction processing. Note that in the dispersion correction processing a predetermined dispersion correction function is acquired.

The processing of step S110 is an example of "dispersion correction processing" of technology disclosed herein.

At step S112, the image processing section 164 obtains a first transform signal by performing a known Inverse Fast Fourier Transform (IFFT) on the dispersion corrected interference spectrum.

At step S114, the image processing section 164 performs a known multi peak detection processing as multi peak detection (MPD) on the post transformation first transform signal. Plural peaks are detected in the multi peak detection processing, and also signal components from a peak value series are detected based on the detected values of plural peaks (peak values).

At step S116, the image processing section 164 saves the plural detected peaks as a peak value series, and also saves the signal components from the peak value series. Namely, data representing a peak value series and signal components from the peak value series is stored in the RAM 16B of the control device 16.

At step S118, the image processing section 164 performs a known fast Fourier transform (FFT) on the saved peak value series, and obtains a second transform signal.

At step S120, the image processing section 164 obtains an inverse dispersion correction signal to perform inverse dispersion correction on the second transform signal from the post-Fourier transform peak value series.

At step S122, the image processing section 164 extracts real part components in the inverse dispersion correction signal. Note that at step S122, the extracted real part components may be multiplied a specific number of times (for example, doubled).

At step S124, the image processing section 164 subtracts the extracted real part components from the acquired interference spectrum. Namely, components of a peak value series (MPD) are subtracted from the original interference spectrum.

The processing of step S124 is an example of processing to obtain a "corrected interference signal" of technology disclosed herein.

At step S126, the image processing section 164 determines whether or not the post-subtraction light intensity I $(=\|r_i\|^2)$ of the interference spectrum is the above threshold th or lower. The determination processing of step S126 may be determination as to whether or not the light intensity of a remaining signal, which is the post-subtraction interference spectrum, conforms to the following Equation (2).

$$\|r_i\|^2 \le E * \frac{\sum_{m=1}^{M}\|f_m\|^2}{M} \quad \cdots (2)$$

In cases in which negative determination is made at step S126, the image processing section 164 sets the post-subtraction interference spectrum as the interference spectrum to be subjected to execution of the above FR process, and then returns to the processing of step S110. However, in cases in which affirmative determination is made at step S126, the image processing section 164 transitions to the processing of step S128.

The processing to subtract a specific value obtained by the above FR process is accordingly executed repeatedly until the interference spectrum from the A-scan becomes the threshold th or lower. The threshold th is an example of a "threshold" of technology disclosed herein.

At step S128, the image processing section 164 determines whether or not the above FR process has been completed on all the A-scans.

In cases in which negative determination is made at step S128, the image processing section 164 returns to the processing of step S108 in order to execute the above FR process on another A-scan, and the present processing routine is ended when affirmative determination is made.

As described above, the signal processing proceeds to step S80 when the step S76 of Fig. 5 is finished.

At step S80, the display control section 166 outputs (displays) OCT data (tomographic image data) on the input/display device 16E. Namely, the data saved at above step S116 is acquired as OCT data, and is output (displayed) on the input/display device 16E.

As described above, in the present exemplary embodiment, the interference spectrum (interference pattern) of each A-scan obtained by respective plural A-scans forming a B-scan image are employed in the full-range OCT processing, and the peak values of light intensity indicated by the interference spectra are extracted. Then the extracted peak value components are subtracted from the original interference spectrum (interference pattern) so as to execute a FR process to remove the mirror signal. The FR process is processing repeated for each A-scan, with the repeated processing executed until the magnitude of the light intensity of the interference spectrum remaining after the peak value components have been subtracted is the predetermined threshold or lower. The predetermined threshold is a specific value derived using above Equation (1), namely, a fixed value determined from the B-scan image is employed as the threshold th. Whether or not to repeat the FR process in the present exemplary embodiment is accordingly determined by a fixed value determined from the threshold th, namely from the image overall obtained by B-scan. This means that because the fixed threshold determined from the B-scan image is common to the FR process for each of the A-scans, uneven brightness appearing as stripes, shadow, or the like is reduced compared to cases in which different thresholds are employed for each of the A-scans, enabling a full-range image of high image quality to be obtained.

Moreover, in an OCT image, uneven brightness related to image quality arises in cases in which the magnitudes of the signals of the images obtained by the respective A-scans are different. For example, if the brightness peak values are different in the signals of plural image obtained from the respective A-scans, then the magnitudes of noise components in the signals are also different. Namely, there is a difference between the magnitudes of the noise components between an A-scan image of a dark region on the fundus and an A-scan image of a bright region thereon. Such noise component differences appear as images (for example, stripes), with uneven brightness arising in the obtained OCT images. In contrast thereto, in the present exemplary embodiment, the determined fixed threshold th is set for all the A-scan images obtained by the B-scan, enabling the brightness of the OCT images to be made more uniform.

The threshold th is, as described above, derived based on a mean value of light intensities of the initial interference patterns (interference spectra) of all the A-scans. Specifically, the threshold th is a value resulting from multiplying the mean value by coefficient E. The coefficient E is predetermined.

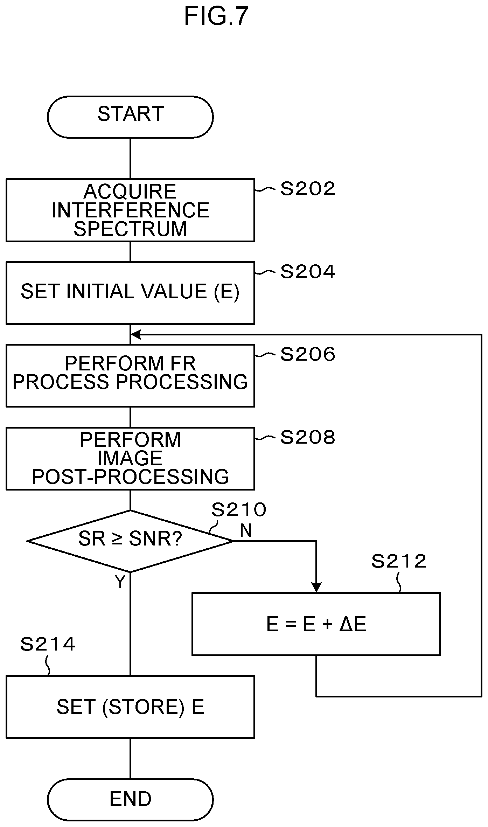

Description follows regarding an example of deriving a ratio of applicable light intensity to remove the above-mentioned mirror image as the coefficient E of threshold th, with reference to Fig. 7.

At step S202, the image processing section 164 acquires an interference spectrum of the examined eye that has already been imaged by the ophthalmic device 110. Note that there is no limitation to an interference spectrum of the examined eye that has already been imaged, and obviously the examined eye may be measured by the ophthalmic device 110, and an interference spectrum stored in the RAM 16B of the control device 16 acquired.

At step S204, the image processing section 164 sets an initial value for coefficient E (for example, E = 0.1).

At step S206, the image processing section 164 executes the FR process to remove the above-mentioned mirror signal (steps S110 to S124 of Fig. 6), and acquires a post-processing image at next step S208. Moreover, the image processing section 164 derives a mirror signal removal ratio SR using the images before and after processing. The mirror signal removal ratio SR may employ a ratio between the pre-processing image signal and the post-processing image signal. The mirror signal removal ratio SR is derived such that the removal ratio is greater as the amount of the mirror signal to remove from the original signal increases. Note that although a case is described above in which the image processing section 164 derives the mirror signal removal ratio SR using the images before and after processing, the image processing section 164 may derive the mirror signal removal ratio SR using only the post-processing image. In such cases, the mirror signal removal ratio SR may employ a signal ratio between the real part and the imaginary part in the post-processing image signal.

At step S210, the image processing section 164 determines whether or not the mirror signal removal ratio SR is greater than or equal to the signal-noise ratio SNR ($SR \geq SNR$). The processing of step S210 corresponds to determination processing as to whether or not a mirror image that is a virtual image has been eliminated from the fundus image. The ratio SNR is predetermined prior to shipping the ophthalmic device 110 according to the imaging target, such as the anterior eye portion or the posterior eye portion, in the ophthalmic device 110. For example, in cases in which a stronger signal (signal with a higher luminosity value) is contained in images imaging the anterior eye portion than in images imaging the posterior eye portion, then good images can be obtained by determining a greater ratio SNR for when imaging the anterior eye portion than when imaging the posterior eye portion. The ratio SNR is accordingly decided according to the imaging target, and the coefficient E derived.

In cases in which negative determination is made at step S210, the image processing section 164 increases the current value of the coefficient E at step S212 by a specific amount ($E = E + \Delta E$), and then processing returns to step S206.

However, in cases in which affirmative determination is made at step S210, at step S214 the image processing section 164 determines the current value of coefficient E as the coefficient E for the ophthalmic device 110 and stores it.

The coefficient E can be pre-determined as described above.

The FR process executed for each A-scan in the full-range OCT processing described above is repeatedly executed until a magnitude of the light intensity of the interference spectrum remaining after subtraction of the peak value components is the threshold or lower. However, repeated processing of the FR process has an effect on a wait time, for example, from the fundus of the examined eye being imaged until the OCT images is displayed. Namely, as the number of times of repeat processing in the FR process for each A-scan increases, the wait time gets longer. Moreover, there is a reduction in convenience of use from the perspective of the operator, such as a doctor or the like, referencing the OCT images due to the increase in the wait time. An upper limit may accordingly be predetermined for the number of repetitions such that the number of times that the FR process is repeated ends at a specific number of times. For example, the wait time may be set to an operator instructed time or to a specific time determined by experimentation, and the processing time on the interference spectrum of each of the A-scans may be determined according to the total number of A-scans such that the processing is ended if the specific time has elapsed since the start of processing. For example, in cases in which the wait time has been set at 5 seconds and the total number of A-scans is 100, then the processing time for the interference spectrum of each of the A-scans is determined as 0.05 seconds (= 5/100), and the upper limit to the number of times of repeated processing may be determined so as not to exceed 0.05 seconds.

Note that although a case has been described in the present exemplary embodiment in which a mean value of light intensities of initial interference patterns of all A-scans is employed as an example of the threshold, technology disclosed herein is not limited to this mean value. For example, a freely selected value intermediate between a maximum value and a minimum value of light intensity may be set as the threshold. Moreover, a weighted average value may be employed, such as a weighted average value with respect to wavelength, or a weighted average value with respect to A-scan position, namely, time. Furthermore, a value computed based on frequency of occurrence, such as on a histogram of light intensities, may be derived as the threshold.

Although a case has been described above in which a mean value of light intensities of initial interference patterns of all A-scans is employed as the threshold th, there is no limitation thereto. For example, an upper limit of noise components in the obtained signal (interference spectrum) or a specific value determined from the noise components may be set as the threshold. For the upper limit of noise components, or the specific value determined from the noise components, a model eye using a reflective surface as a retina instead of the examined eye may be employed, and a signal containing electrical noise obtained when light hits the retina portion of the model eye may be determined as the threshold.

Moreover, the threshold may be determined utilizing a past B-scan image. For example, when such imaging is performed, the threshold may be computed from a B-scan image obtained at previous imaging.

Furthermore, the threshold th may employ a value derived using a machine learning model trained using interference spectra from which mirror images have been removed as training signals, input with an interference spectrum containing a mirror image as the input signal and outputting the threshold as the output signal.

In the above signal processing (full-range OCT processing), a light intensity to subtract from an original image in order to remove a signal appearing as a mirror image is adjusted by setting based on the light intensity of all A-scans with the number of repetitions of the FR process set for each of the A-scans. This thereby enables mirror image signal components of appearing as a complex conjugate component image to be removed from tomographic images more efficiently than cases in which the processing of the FR process is repeated the same number of times for all of the A-scans.

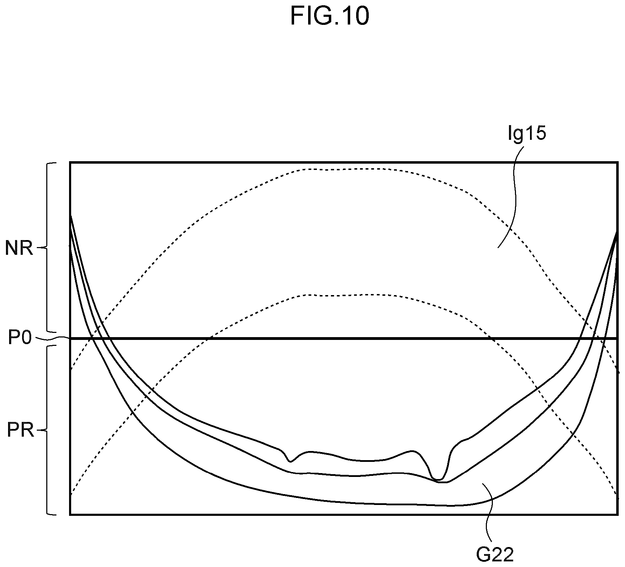

Fig. 10 illustrates an example of an OCT image (tomographic image) from normal full-range OCT processing without execution of the above signal processing. As illustrated in Fig. 10, the mirror image Ig15 is present in the tomographic image prior to mirror image removal. Moreover, an artifact appearing as stripes in the tomographic image is sometimes generated when, for example, a fluctuation in brightness occurs between adjacent A-scans in the tomographic image.

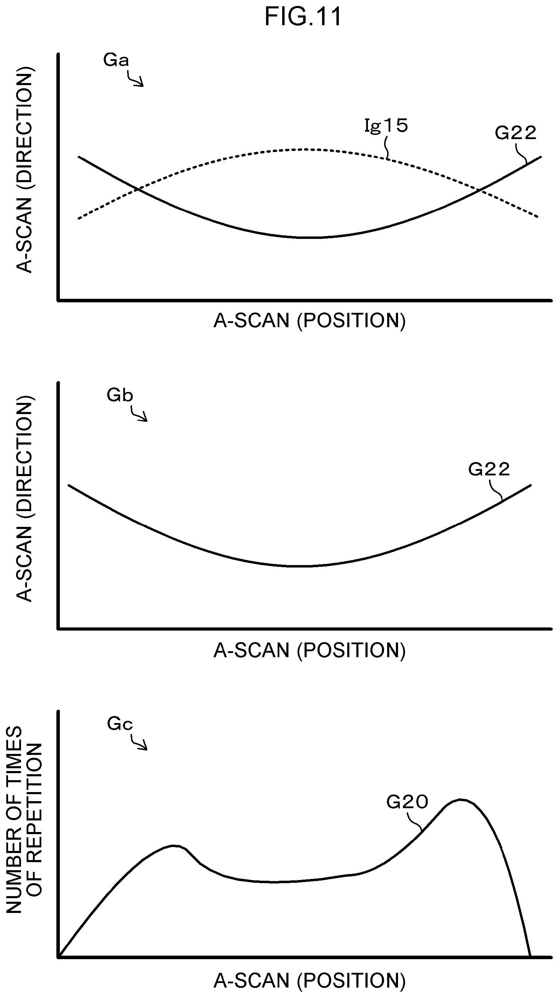

Fig. 11 illustrates a conceptual diagram related to an OCT image (tomographic image) from full-range OCT processing. Fig. 11 includes images of an OCT image Ga, an OCT image Gb, and includes a FR process repeat number of times distribution Gc.

The OCT image Ga is an OCT image from normal full-range OCT processing without execution of the above signal processing. In the OCT image Ga, an image G22 representing a fundus portion is illustrated by a solid line, and a mirror image Ig 15 is illustrated conceptually by a broken line. The OCT image Gb is an OCT image by the full-range OCT processing of the present exemplary embodiment. The mirror image Ig 15 is removed in the OCT image Gb, and only the image G22 representing the fundus portion is contained therein. Furthermore, the FR process repeat number of times distribution Gc is a distribution of the repeat number of times that the FR process is repeated on each A-scan in the full-range OCT processing of the present exemplary embodiment. In the OCT image Gc, a repeat number of times distribution characteristic G20 is illustrated conceptually by a solid line. As indicated by the distribution characteristic G20, the repeat number of times of FR process is changed for each of the A-scans. This is because a fixed value is employed as the threshold th, and this fixed value is employed as a determination benchmark for whether or not to repeat the FR process.

Increases and decreases in the repeat number of times of the FR process performed to remove the mirror image affect the mirror image appearing in the post-processing OCT image, and affect the brightness and image quality of the OCT image.

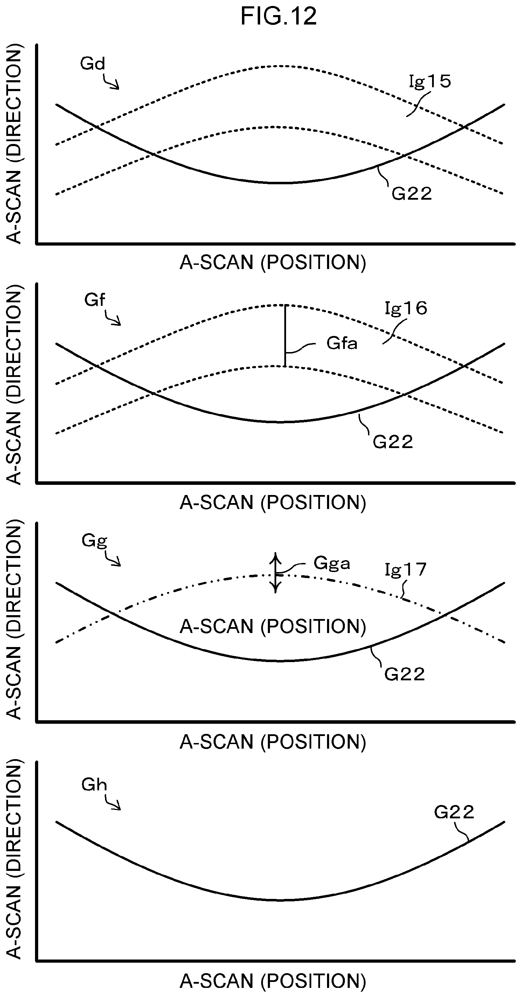

Fig. 12 illustrates a conceptual diagram related to an OCT image (tomographic image) by the full-range OCT processing as it changes according to the repeat number of times of FR process. Fig. 12 includes each diagram of an OCT image Gd, an OCT image Gf, an OCT image Gg, and an OCT image Gh in which the signal processing of the present exemplary embodiment has been executed.

The OCT image Gd is a conceptualized image of Fig. 10, and is an OCT image from normal full-range OCT processing. The OCT image Gd may be thought of as being an OCT image obtained by signal processing without the repetition of the signal processing of the present exemplary embodiment.

The OCT image Gf, the OCT image Gg, and the OCT image Gh are OCT images obtained as a result of signal processing with different repeat number of times of the FR process. The OCT image Gf is an OCT image by full-range OCT processing obtained by full-range OCT processing in which the number of repetitions of the FR process is reduced by using a threshold greater than the threshold than the threshold th described above. In the OCT image Gf, the above described peak values remain and increase as the FR process repeat number of times gets fewer, and the mirror image Ig16 generated as stripes Gfa is contained therein. This means that uneven brightness arises in the OCT image, and the image quality decreases.

The OCT image Gg is an OCT image from full-range OCT processing in which a larger FR process repeat number of times is set by using a smaller threshold than the threshold th above. The OCT image Gg is an image in which removal of the mirror image proceeds as the FR process repeat number of times increases, and the light intensity is only reduced at the position of the mirror image and contains a shadow image Ig17 where the stripe Gga of lower light intensity than other regions is contiguous. This means that the image G22 illustrating the fundus portion, the image of the background to image 22 and the shadow image Ig17 are perceived by the operator, and so although the mirror image itself is not recognized, the position and region of the mirror image are recognized from the shadow thereof. This means that uneven brightness is generated in the OCT image, and the image quality is reduced.

In contrast thereto, in the OCT image Gh executed with the signal processing of the present exemplary embodiment, similarly to in the OCT image Gb illustrated in Fig. 11, the mirror image Ig15 is removed without stripes or shadow arising as described above, and only the image G22 representing the fundus portion is contained therein. Namely, the FR process is repeated in the present exemplary embodiment for a suitable number of repetitions that conforms to the signal obtained by each A-scan using the fixed value threshold th determined from the B-scan image as the determination benchmark. This accordingly enables uneven brightness in the OCT image to be reduced, enabling a high image quality OCT image to be provided.

The above signal processing is performed in this manner in the present exemplary embodiment, namely the peak values of light intensity are extracted from the interference spectrum (interference pattern) of each of the A-scans, and components of the extracted peak values are subtracted from the original interference spectrum (interference pattern). This thereby enables the signal components of the mirror image appearing as a complex conjugate component image in the tomographic image to be removed. This means that, as illustrated in Fig. 9, the mirror image can be removed with good accuracy from the tomographic image, enabling artifacts to be suppressed from appearing in the tomographic image. This thereby enables the emergence of artifacts in the tomographic image displayed in full-range to be suppressed from occurring in the present exemplary embodiment, and enables a tomographic image accurately reflecting content of the fundus to be obtained.

The OCT data (tomographic image data) is transmitted to the management server 140 via the network 130, and is stored in the memory of the management server 140 associated with the patient ID of each patient. The management server 140 transmits patient data together with OCT data (tomographic image data) via the network 130 under instruction from the image viewer 150. The image viewer 150 displays patient data together with an OCT image on the display screen of a display.

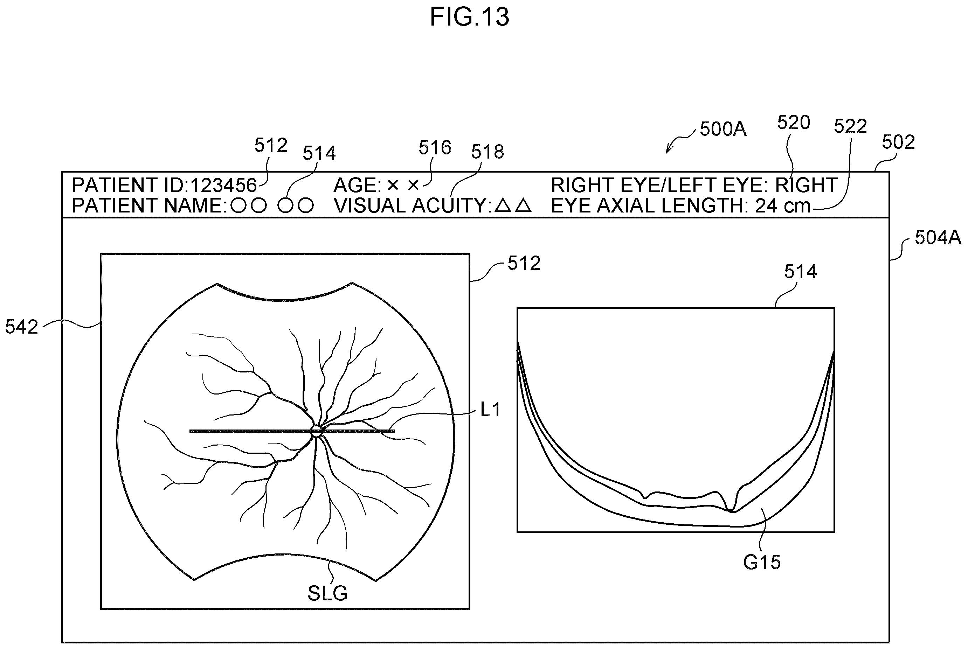

Description follows regarding a display-screen to display patient data together with OCT data (tomographic image data) on the display screen of the display of the image viewer 150. Fig. 13 illustrates a display-screen 500A. As illustrated in Fig. 13, the display-screen 500A includes an information area 502, and an image display area 504A.

The information area 502 includes a patient ID display field 512, a patient name display field 514, an age display field 516, a visual acuity display field 518, a right eye/left eye display field 520, and an eye axial length display field 522. Based on the information received from the management server 140, the image viewer 150 displays various information in each of the respective display regions from the patient ID display field 512 to the eye axial length display field 522.

The image display area 504A is region for displaying an examined eye image or the like. Each of the following display fields is provided in the image display area 504A, specifically an OCT image display field 540 and a UWF fundus image display field 542.

A comment field may also be provided in the image display area 504A. The comment field is a remark field for free input with observed results or diagnostic results by an ophthalmologist who is a user.

A UWF-SLO fundus image SLG imaging the fundus of the examined eye with the ophthalmic device 110 is displayed in the UWF fundus image display field 542. A range where the tomographic image was acquired is, for example, a range L1 overlap-displayed on the UWF-SLO fundus image SLG.

The OCT image is displayed in the OCT image display field 540. In Fig. 13, a tomographic image G15 based on the OCT data obtained by executing full-range processing is displayed. Note that when the OCT data (tomographic image data) is transmitted to the management server 140 and the image viewer 150, data indicating whether full-range OCT processing was executed or normal OCT processing was executed is also transmitted. In order to display a tomographic image, based on this data determination is made as to whether or not the tomographic image is to be displayed in full-range, and then the tomographic image is displayed either in full-range or normal range according to the determination result.

Although in the present exemplary embodiment the signal processing program is executed by the CPU 16A of the ophthalmic device 110, technology disclosed herein is not limited thereto and, for example, the signal processing program may be executed by a CPU of the management server 140 or the image viewer 150.

In the present disclosure, each of the configuration elements (devices and the like) may be present singly or present as two or more thereof as long as inconsistencies do not result therefrom.

Each example described above is an example of a case in which image processing is implemented by a software configuration using a computer, however technology disclosed herein is not limited thereto. For example, the image processing may be executed by a hardware configuration alone, such as a field programmable gate array (FPGA), an application specific integrated circuit (ASIC), or the like, instead of the software configuration using the computer. Part of the processing from out of the image processing may be executed by a software configuration, and the remaining processing may be executed by a hardware configuration.

In technology disclosed herein, the image processing includes cases implemented by a software configuration using a computer, cases implemented by a hardware configuration, and cases implemented by a configuration that combines a software configuration and a hardware configuration. The technology disclosed herein includes the following technologies.

First Technology

An ophthalmic device including:

an interference signal acquisition section configured to obtain an interference signal from interference light between measurement light obtained by scanning an examined eye with light from a light source and reference light of light divided from the light source;

a component acquisition section configured to acquire a peak value component determined by a light intensity distribution in the interference signal;

a corrected interference signal acquisition section configured to obtain a corrected interference signal by subtracting a signal of magnitude determined from the peak value component from the interference signal;

a repeat execution section configured to employ the corrected interference signal as the interference signal, and repeatedly execute the step of obtaining the peak value component for the interference signal and the step of obtaining the corrected interference signal until a light intensity of the subtraction result corrected interference signal is a predetermined threshold or lower; and
an OCT signal acquisition section configured to acquire OCT data based on the corrected interference signal of the threshold or lower.

Second Technology

A signal processing method of an ophthalmic device, including:

a step in which an interference signal acquisition section obtains an interference signal from interference light between measurement light obtained by scanning an examined eye with light from a light source and reference light of light divided from the light source;
a step in which a component acquisition section obtains a peak value component determined by a light intensity distribution in the interference signal;
a step in which a corrected interference signal acquisition section obtains a corrected interference signal by subtracting a signal of magnitude determined from the peak value component from the interference signal;
a step in which a repeat execution section employs the corrected interference signal as the interference signal, and repeatedly executes the step of obtaining the peak value component for the interference signal and the step of obtaining the corrected interference signal until a light intensity of the subtraction result corrected interference signal is a predetermined threshold or lower; and
a step in which an OCT signal acquisition section obtains OCT data based on the corrected interference signal of the threshold or lower.

The following technology is proposed from the above disclosed content.

Third Technology

A computer program product for obtaining an OCT signal, the computer program product includes a computer-readable storage medium that is not in itself a transitory signal, with the program stored on the computer-readable storage medium and the program executing processing on a computer including:

a step of obtaining an interference signal from interference light between measurement light obtained by scanning an examined eye with light from a light source and reference light of light divided from the light source;
a step of obtaining a peak value component determined by a light intensity distribution in the interference signal;
a step of obtaining a corrected interference signal by subtracting a signal of magnitude determined from the peak value component from the interference signal;
a step of employing the corrected interference signal as the interference signal, and repeatedly executing the step of obtaining the peak value component for the interference signal and the step of obtaining the corrected interference signal until a light intensity of the subtraction result corrected interference signal is a predetermined threshold or lower; and
a step of obtaining OCT data based on the corrected interference signal of the threshold or lower.

The signal processing described above is merely an example thereof. Accordingly, redundant steps may be omitted, new steps may be added, and the processing sequence may be swapped around within a range not departing from the spirit of technology disclosed herein.
All publications, patent applications and technical standards mentioned in the present specification are incorporated by reference in the present specification to the same extent as if each individual publication, patent application, or technical standard was specifically and individually indicated to be incorporated by reference. Moreover, the entire content of the disclosure of Japanese Patent Application No. 2023-159176 filed on September 22, 2023 is incorporated by reference in the present specification.

**Claims**

1. An ophthalmic device including a processor, wherein the processor executes processing comprising:

a step of obtaining an interference signal from interference light between measurement light obtained by scanning an examined eye with light from a light source and reference light of light divided from the light source;
a step of obtaining a peak value component determined by a light intensity distribution in the interference signal;

a step of obtaining a corrected interference signal by subtracting a signal of magnitude determined from the peak value component from the interference signal;

a step of employing the corrected interference signal as the interference signal, and repeatedly executing the step of obtaining the peak value component for the interference signal and the step of obtaining the corrected interference signal until a light intensity of the subtraction result corrected interference signal is a predetermined threshold or lower; and

a step of obtaining OCT data based on the corrected interference signal of the threshold or lower.

2. The ophthalmic device of claim 1, wherein the threshold is a value determined based on a light intensity distribution of a plurality of the interference signals from scanning an examined eye in a depth direction for a predetermined range scanned in a direction orthogonal to the depth direction.

3. The ophthalmic device of claim 2, wherein the threshold is a value determined based on a mean value of light intensity of interference signal obtained by the step of obtaining an interference signal from the interference light.

4. The ophthalmic device of claim 2, wherein the threshold is a value determined by multiplying a value determined based on a light intensity distribution of the plurality of interference signals by multiplication with a predetermined specific ratio.

5. The ophthalmic device of claim 2, wherein:

the threshold is a value determined by a condition equation represented by:

$$\mathrm{E} * \frac{\sum_{m=1}^{M} \|f_m\|^2}{M}$$

wherein f is an initial light intensity in an interference signal acquired by scanning in a depth direction, M is a total number of scans in the depth direction included in scanning of a specific range in a direction orthogonal to the depth direction, and E is a specific coefficient.

6. The ophthalmic device of any one of claim 1 to claim 5, wherein the repeated execution step is stopped when a predetermined specific time has elapsed from step start.

7. The ophthalmic device of any one of claim 1 to claim 6, wherein:

the processing further comprises a step of performing dispersion correction processing on the interference signal; and

the signal resulting from performing the dispersion correction processing is employed as the interference signal.

8. The ophthalmic device of any one of claim 1 to claim 7, wherein:
the processing further comprises a step of outputting an image obtained based on the OCT data to a display screen over an output range of an image in a depth direction of a fundus with reference to a position corresponding to a light path length of the reference light and in an opposite direction to the depth direction.

9. A signal processing method performed by a processor of an ophthalmic device, the signal processing method comprising:

a step of obtaining an interference signal from interference light between measurement light obtained by scanning an examined eye with light from a light source and reference light of light divided from the light source;

a step of obtaining a peak value component determined by a light intensity distribution in the interference signal;

a step of obtaining a corrected interference signal by subtracting a signal of magnitude determined from the peak value component from the interference signal;

a step of employing the corrected interference signal as the interference signal, and repeatedly executing the step of obtaining the peak value component for the interference signal and the step of obtaining the corrected interference signal until a light intensity of the subtraction result corrected interference signal is a predetermined threshold or lower; and

a step of obtaining OCT data based on the corrected interference signal of the threshold or lower.

10. A program that causes processing to be executed on a computer, the processing comprising:

a step of obtaining an interference signal from interference light between measurement light obtained by scanning an examined eye with light from a light source and reference light of light divided from the light source;

a step of obtaining a peak value component determined by a light intensity distribution in the interference signal;

a step of obtaining a corrected interference signal by subtracting a signal of magnitude determined from the peak value component from the interference signal;

a step of employing the corrected interference signal as the interference signal, and repeatedly executing the step of obtaining the peak value component for the interference signal and the step of obtaining the corrected interference signal until a light intensity of the subtraction result corrected interference signal is a predetermined threshold or lower; and

a step of obtaining OCT data based on the corrected interference signal of the threshold or lower.

EP 4 781 895 A1

# FIG.1

16

# FIG.2

EP 4 781 895 A1

FIG.3

19 IMAGING OPTICAL SYSTEM

20P

56

58

54

52 LIGHT SOURCE

60

64 A/D CONVERTER

62 DETECTOR

16 CONTROL DEVICE

## FIG.4

16A

IMAGING CONTROL SECTION — 162

IMAGE PROCESSING SECTION — 164

DISPLAY CONTROL SECTION — 166

# FIG.5

START

S72 — INSTRUCT OCT SCAN

S74 — FULL-RANGE SET? — N

Y

S76 — PERFORM FULL-RANGE OCT PROCESSING

S78 — PERFORM NORMAL OCT PROCESSING

S80 — OUTPUT OCT DATA (TOMOGRAPHIC IMAGE DATA)

END

# FIG.6

```
                    START
                      │
S102 ── ACQUIRE INTERFERENCE
         SPECTRUM
                      │
S104 ── PERFORM RE-SAMPLING,        ┌──────────────────────────────┐
         ZERO PADDING, WINDOW       │ PERFORM DISPERSION     S110  │
         FUNCTION PROCESSING        │ CORRECTION                   │
                      │             ├──────────────────────────────┤
S106 ── COMPUTE MEAN VALUES OF      │ PERFORM INVERSE FAST   S112  │
         LIGHT INTENSITIES OF INITIAL│ FOURIER TRANSFORM (IFFT)    │
         INTERFERENCE PATTERN       ├──────────────────────────────┤
         OF ALL A-SCANS AND         │ PERFORM MULTI PEAK     S114  │
         COMPUTE THRESHOLD          │ DETECTION (MPD)              │
                      │             ├──────────────────────────────┤
         INSTRUCT A-SCAN            │ SAVE DETECTED          S116  │
                                    │ MULTI PEAK VALUES            │
         S108                       ├──────────────────────────────┤
                                    │ PERFORM FAST FOURIER   S118  │
                                    │ TRANSFORM (FFT)              │
                                    ├──────────────────────────────┤
                                    │ PERFORM INVERSE        S120  │
                                    │ DISPERSION CORRECTION        │
                                    ├──────────────────────────────┤
                                    │ EXTRACT REAL PART      S122  │
                                    ├──────────────────────────────┤
                                    │ SUBTRACT MPD           S124  │
                                    │ COMPONENTS FROM              │
                                    │ INTERFERENCE SPECTRUM        │
                                    └──────────────────────────────┘
```

S126 — LIGHT INTENSITY I ≤ TH — N

S128 — ALL A-SCANS COMPLETED? — N

END

# FIG.7

```
        ┌──────────────┐
        │    START     │
        └──────┬───────┘
               │
    ┌──────────────────────┐  S202
    │       ACQUIRE        │
    │     INTERFERENCE     │
    │      SPECTRUM        │
    └──────────┬───────────┘
               │
    ┌──────────────────────┐  S204
    │  SET INITIAL VALUE (E)│
    └──────────┬───────────┘
               │
    ┌──────────────────────┐  S206
    │    PERFORM FR        │
    │  PROCESS PROCESSING  │
    └──────────┬───────────┘
               │
    ┌──────────────────────┐  S208
    │      PERFORM         │
    │       IMAGE          │
    │   POST-PROCESSING    │
    └──────────┬───────────┘
               │          S210
          ◇ SR ≥ SNR? ◇────N──────┐
               │                   │
               Y            ┌──────────────┐  S212
               │            │  E = E + ΔE  │
    ┌──────────────────────┐└──────────────┘
    │    SET (STORE) E     │  S214
    └──────────┬───────────┘
               │
        ┌──────────────┐
        │     END      │
        └──────────────┘
```

# FIG.8

EP 4 781 895 A1

# FIG.9

NR

P0

PR

G15

EP 4 781 895 A1

# FIG.10

# FIG.11

Ga

Ig15    G22

A-SCAN (DIRECTION)

A-SCAN (POSITION)

Gb

G22

A-SCAN (DIRECTION)

A-SCAN (POSITION)

Gc

G20

NUMBER OF TIMES OF REPETITION

A-SCAN (POSITION)

# FIG.12

## FIG.13

PATIENT ID:123456    AGE: × ×    RIGHT EYE/LEFT EYE: RIGHT

PATIENT NAME:○○ ○○    VISUAL ACUITY:△△    EYE AXIAL LENGTH: 24 cm

L1

SLG

G15

EP 4 781 895 A1

# INTERNATIONAL SEARCH REPORT

| International application No. |
| --- |
| **PCT/JP2024/032581** |

| **A.** | **CLASSIFICATION OF SUBJECT MATTER** |
| --- | --- |

*A61B 3/10*(2006.01)i; *G01N 21/17*(2006.01)i
FI:  A61B3/10 100; G01N21/17 630

According to International Patent Classification (IPC) or to both national classification and IPC

| **B.** | **FIELDS SEARCHED** |
| --- | --- |

Minimum documentation searched (classification system followed by classification symbols)

A61B3/10; G01N21/17

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| A | JP 2023-518174 A (LEICA MICROSYSTEMS INC.) 28 April 2023 (2023-04-28) entire text, all drawings | 1-10 |
| A | JP 2019-33956 A (CANON KABUSHIKI KAISHA) 07 March 2019 (2019-03-07) entire text, all drawings | 1-10 |
| A | JP 2006-122649 A (NIDEK KK) 18 May 2006 (2006-05-18) entire text, all drawings | 1-10 |
| A | US 2012/0026462 A1 (UHLHORN, Stephen) 02 February 2012 (2012-02-02) entire text, all drawings | 1-10 |
| A | US 2012/0188555 A1 (IZATT, Joseph A.) 26 July 2012 (2012-07-26) entire text, all drawings | 1-10 |
| P, X | JP 2024-21390 A (TOMEY CORPORATION) 16 February 2024 (2024-02-16) paragraphs [0009]-[0013], [0046]-[0074], fig. 1-11 | 1, 7-10 |
| P, A | | 2-6 |

| ☐ Further documents are listed in the continuation of Box C. | ☑ See patent family annex. |
| --- | --- |

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **29 October 2024** | **12 November 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/032581**

| Patent document cited in search report | | | Publication date (day/month/year) | Patent family member(s) | | | Publication date (day/month/year) |
|---|---|---|---|---|---|---|---|
| JP | 2023-518174 | A | 28 April 2023 | WO | 2021/183499 | A1 | |
| | | | | US | 2023/0148858 | A1 | |
| | | | | CN | 115551404 | A | |
| JP | 2019-33956 | A | 07 March 2019 | WO | 2019/035426 | A1 | |
| JP | 2006-122649 | A | 18 May 2006 | US | 2006/0087616 | A1 | |
| US | 2012/0026462 | A1 | 02 February 2012 | US | 2012/0140173 | A1 | |
| US | 2012/0188555 | A1 | 26 July 2012 | WO | 2012/100213 | A2 | |
| JP | 2024-21390 | A | 16 February 2024 | US | 2024/0053137 | A1 | |
| | | | | paragraphs [0026]-[0030], [0055]-[0089], fig. 1-11 | | | |
| | | | | EP | 4317909 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200037872 A
- JP 2023159176 A